# EUROPEAN PATENT APPLICATION

(11) **EP 4 643 829 A1**
(43) Date of publication of application: **05.11.2025**
(21) Application number: 24173250.2
(22) Date of filing: 30.04.2024
(51) Int. Cl.: A61F 5/01

(54) **HINGE MECHANISM FOR A KNEE ORTHOSIS, PROCEDURE FOR FITTING A HINGE MECHANISM, AND A KNEE ORTHOSIS**

(71) Applicant: Orthopedie De Rycker BV, 9000 Gent (BE)
(72) Inventor: De Rycker, Tom, 9830 Sint-Martens-Latem (BE)
(74) Representative: De Clercq & Partners

(57) **Abstract**

The invention is related to the technical field of orthopedic devices, in particular knee orthoses for supporting a knee joint, and more specifically of an orthopedic device with a hinge, wherein the movement of the device can be limited by means of the hinge and where the hinge of the device is formed via 3D printing so that a knee orthosis can be made to measure for the patient and where a minimum of different parts is required for the assembly of the knee brace. The invention also includes a method for installing such hinge mechanisms and a knee orthosis provided with such hinge mechanisms.

## Description

### Technical Domain

The invention is related to the technical field of orthopedic devices, in particular knee orthoses for supporting a knee joint, and more specifically on an orthopedic device with a hinge, wherein the movement of the device can be limited by means of the hinge and where the hinge of the device is formed via 3D printing, such that a knee orthosis can be made tailored to the patient, and wherein a minimum of different parts is required for the assembly of the knee brace. The invention also includes a method for installing such hinge mechanisms and a knee orthosis provided with such hinge mechanisms.

### Prior art

Knee orthoses are used to stabilize, immobilize or support the knee in various types of injuries such as torn cruciate ligaments or knee osteoarthritis or to aid recovery after knee surgery. For example, an untreated injury to the knee joint or the cruciate ligaments of the knee joint will have a prolonged healing process, since in such cases, the knee joint is often unstable. To prevent or limit this instability of the knee joint, knee orthoses or knee braces are used to treat such injuries and prevent further injury. Various types of knee orthoses such as semi-rigid, rigid or flexible knee braces are already known, and depending on the specific situation they can support the movement of the knee, absorb the pressure and/or weight of the patient's body, or perform a controlling function. Iin the prior art prefabricated ortheses are already known, wherein the clamping around the knee can be adjusted, for example, by means of a velcro strap that is attached around the upper leg and lower leg, so that these prefabricated orthoses can be used for a large group of patients and are therefore less expensive to purchase. Although the orthosis itself can already be adjusted to the individual patient's size, the hinge and housing around the leg itself is an *offthe-shelf product,* meaning that the ability to adjust the orthesis is limited by these components.

Moreover, not every hinge is suitable for every application. For example, a passive single-axis hinge will only be useful when the forces transmitted to the tibia are constant throughout the full flexion angle, while a damping hinge will reduce sagging by preventing sudden rotation of the knee. Knee braces with a *4-bar hinge design* generally use two hinges that limit, prevent and/or guide the movement of the knee joint. These hinges, positioned to the left and right of the knee respectively, can be provided with coupling points to create a 4-bar system. Such 4-bar systems are constructed in such a way that the four connection points together determine the freedom of movement of the knee brace. Strategic placement of the four connection points will allow the knee to move in specific ways, while the stability of the knee remains guaranteed and stressful or damaging movements can be prevented.

Although several types of hinges are already known, certain types of hinges may be unsatisfactory in the treatment of certain injuries, as they can, for example, cause the patient to use muscles for leg movement, which in turn can lead to an unwanted lengthening of, for example, the cruciate ligaments. In the case of a knee brace that is equipped with a hinge with a 4-bar design, a number of pre-placed openings are provided in the hinge, in which these 4 coupling pieces can be mounted. By using these specific openings, certain movements can be permitted, while other freedoms of movement are not possible. Since this type of hinge is an *off-the-shelf* product, the options for placing the connectors are limited and the adaptability to the needs of the individual patient is limited.

There is therefore a need for knee orthoses where the entire orthesis, including the hinge, can be made to measure for the patient and according to his or her needs, using 3D printing. In addition, there is a need for a hinge for such knee orthoses that accurately imitates the center of rotation behavior or Instant Center of Rotation (ICoR) of the biological knee, providing a dynamic load during bending and extension of the leg, which can prevent strain on the cruciate ligaments. Moreover, there is a need for such a knee orthosis to be quickly available for use by the patient, to be light in weight, and where all parts can be fully customized to suit the patient's leg in order to increase wearing comfort and thus to provide an orthosis that fully meets the needs of the patient, while the cost price for custom-making the knee orthosis remains low, in contrast to traditional production methods for custom-made orthoses.

### Summary

According to one aspect, a hinge mechanism for a knee orthosis is provided, wherein the hinge mechanism consists of a first part preferably permanently connected to an upper part, wherein the upper part can be placed above a knee joint, a second part preferably permanently connected to a lower part, wherein the lower part can be placed under the knee j oint, a first coupling piece, hinge-connected to the first part and the second part, and a second coupling piece, hinge-connected to the first part and the second part (20). The first part and the second part are provided with incisions, wherein the first coupling piece is inserted into the incision on a first side and hinge-attached to the first part, and wherein the coupling piece is inserted into the incision on the other side and hinge-attached to the second part. By using incisions in the first and second part, it is possible to insert at least one coupling piece into these incisions and thus obtain a compact assembly without sacrificing functionality.

According to an embodiment, a hinge mechanism is provided, wherein the hinge mechanism further consists of a cover plate and wherein the second coupling piece is positioned between the cover plate on the one hand, and the first part and the second part on the other hand.

By using a cover plate it is possible to shield the rotating parts of the hinge mechanism from the environment so that when the hinge mechanism operates, the movement cannot be hindered by, for example, clothing worn by the patient.

According to an embodiment, the cover plate is permanently connected to the first part. Because the cover plate is only permanently connected to one part, the cover plate will move with the hinge movement. In this way, the moving parts of the hinge mechanism will always be covered, regardless of the relative position of the first part relative to the second part.

According to an embodiment, the cover plate is provided with at least one bulge and the first part is provided with at least one recess so that, after mounting the cover plate on the first part, the at least one bulge of the cover plate engages in the at least one recess. of the first part.

By using at least one bulge and recess, it is possible to achieve correct alignment of the cover plate with respect to the first part during installation. Moreover, by using a bulge and recess, the cover plate will move in a controlled manner with the hinging movement of the first part. Even if the cover plate is not sufficiently secured to the first part, the cover plate will always move correctly with the first part due to the intervention of the at least one protrusion in the at least one recess.

According to an embodiment, the at least one protrusion has a wedge shape and the at least one recess has a complementary wedge-shaped opening. By using a protrusion with a wedge shape and a recess with a complementary wedge-shaped opening, it is possible to securely attach the cover plate to the hinge mechanism.

According to an embodiment, a hinge mechanism is provided, wherein the first part is positioned relative to the second part such that the first side of the second part is flat with respect to the first side of the first part. By using the incisions, it is possible to insert a coupling piece into these incisions and connect the coupling piece to the first and second part. In this way it is possible to position the first and second parts relative to each other in such a way that one side each of the first and second parts come to lie flat against each other. This makes it possible to assemble a compact but robust and functional hinge mechanism.

According to an embodiment, a hinge mechanism is provided, wherein the top of the second part is rounded, and wherein the first part is provided with a rounding that is complementary to the rounded top. This makes it possible to enable guidance of the first part relative to the second part during the hinging movement of the hinge mechanism.

According to an embodiment, the incision is provided in the rounded top of the second part.

According to one embodiment, a hinge mechanism is provided, wherein the first coupling piece and the second coupling piece are hingedly connected to the first part and the second part by means of a bolt and a nut. According to an embodiment, the bolt is provided with screw threading over a first length and is flat over a second length.

By using such a bolt and nut, the coupling pieces are still arranged movably relative to the first and second parts of the hinge mechanism.

According to an embodiment, a hinge mechanism is provided, wherein the second side of the first part and the second side of the second part are provided with recesses, and wherein the recess is complementary to the shape of the nut. In this way it is possible to lock the nut in the recess provided in the rear of the first or second part, thus allowing a hinging movement without the bolt and nut unscrewing from each other.

According to a further aspect, a method for mounting the hinge mechanism according to any of the preceding claims is provided, the method consisting of the following steps:
- inserting the first coupling piece into the incision of the first part,
- hinged attachment of the first coupling piece to the first part by means of fastening means,
- hinged attachment of the second coupling piece to the second part by means of fastening means,
- inserting the first coupling piece into the incision of the second part,
- hinged attachment of the first coupling piece to the second part by means of a fastening means,
- hinged attachment of the first coupling piece to the second part by means of a fastening means.

By using the incisions it is possible to obtain a compact assembly of a hinge mechanism using a limited number of parts.

According to an embodiment, a method is further provided, the method further comprising the steps:
- positioning at least one protrusion of a cover plate relative to at least one complementary recess in the first part; and
- connecting the cover plate to the first part by means of a fastening means.

According to a next aspect, a knee orthosis is provided, wherein the knee orthosis is provided with two hinge mechanisms and wherein the first hinge mechanism is positioned on a first side of the knee orthosis between the upper part and the lower part of the knee orthosis, and wherein the second hinge mechanism is positioned on a second side of the knee orthosis between the upper part and the lower part of the knee orthosis.

According to one embodiment, a knee orthosis is provided wherein the first part of the first hinge mechanism, the upper part and the first part of the second hinge mechanism are formed in one piece, and wherein the second part of the first hinge mechanism, the lower part and the second part of the second hinge mechanism are formed in one piece.

According to an embodiment, a knee orthosis is provided in which the parts of the knee orthosis are formed via 3D printing.

### Figures

By way of example, a number of embodiments will be described with reference to the following figures:
- **Figure 1** shows a 3D side view of the hinge mechanism according to the invention;
- **Figure 2** shows a side view of the hinge mechanism shown in Figure 1;
- **Figure 3** shows a front view of the hinge mechanism shown in Figure 1;
- **Figure 4** shows a side view of a first part of the hinge mechanism that can be connected to an upper part of a knee orthosis;
- **Figure 5** shows a side view of a second part of the hinge mechanism that can be connected to an upper part of a knee orthosis;
- **Figure 6** shows a 3D side view of the first part as shown in Figure 4;
- **Figure 7** shows a 3D side view of the second part as shown in Figure 5;
- **Figure 8A** shows a side view of a first coupling piece of the hinge mechanism shown in Figure 1;
- **Figure 8B** shows a side view of a second coupling piece of the hinge mechanism shown in Figure 1;
- **Figure 9A** shows a 3D side view of a cover plate of the hinge mechanism shown in Figure 1;
- **Figure 9B** shows a 3D side view of the cover plates shown in Figure 9A;
- **Figure 10** shows the exploded view of the hinge mechanism shown in Figure 1;
- **Figure 11** shows the exploded view of the hinge mechanism shown in Figure 1, including the cover plate shown in Figures 9A and 9B;
- **Figure 12A** shows a 3D side view of the second part of the hinge mechanism connected to the lower part of a knee orthosis;
- **Figure 12B** shows a 3D side view of an alternative as shown in Figure 12A;
- **Figure 13** shows a 3D side view of the first part of the hinge mechanism connected to the lower part of a knee orthosis;
- **Figure 14** shows a 3D representation of a knee orthosis, wherein the first part and the second part of the hinge mechanism are positioned relative to each other;
- **Figure 15** shows a side view of the knee orthosis shown in Figure 14;
- **Figure 16** shows another side view of the knee orthosis shown in Figure 14; and
- **Figure 17** shows a front view of the knee orthosis shown in Figure 14;

### Description

Before describing the invention, it should be understood that this invention is not limited to specific systems and methods or combinations described, as such systems and methods and combinations may of course vary. It should also be understood that the terminology used herein is not intended to be limiting, as the scope of the present invention is limited only by the appended claims.

As further used in this text, the singular forms "a", "the", "the" include both the singular and the plural unless the context is clearly different.

The terms "comprise", "comprises" as further used are synonymous with "inclusive", "include" or "contain", "contains" and are inclusive or open and do not exclude additional, unnamed members, elements or working methods steps. The terms "comprise" and "comprises" shall include the term "contain" and vice versa.

The enumeration of numerical values using a range of numbers includes all values and fractions in these ranges, as well as the quoted endpoints.

The terms "approximately", "essentially", "principally", and the like, as used when referring to a measurable value such as a parameter, a quantity, a duration, and so on, are intended to cover variations of +/- 10% or less, preferably +/-5% or less, more preferably +/-1% or less, and more preferably +/-0.1% or less, from and above the specified value, to the extent that the variations apply to function in the invention described herein. It should be understood that the value referred to by the term "approximately" itself has also been disclosed.

In the following passages various aspects of the invention are further defined. Any aspect so defined may be combined with any other aspect or aspects unless clearly stated to the contrary. In particular, a feature referred to as "preferred" or "beneficial" may be combined with other features or properties stated as "preferred" and/or "beneficial." In this specification, reference to "one embodiment" or "an embodiment" means that a particular function, structure or characteristic described in connection with the embodiment is applicable in at least one embodiment of the invention in question. Where the phrases "in one embodiment" or "an embodiment" appear at different places in this specification, they do not necessarily refer to the same embodiment, although this is not excluded. Furthermore, the features, structures or characteristics described may be combined in any suitable manner, as will be apparent to one skilled in the art from this description. The embodiments described and claimed in the claims can be used in any combination. In the present description of the invention, reference is made to the accompanying drawings, which form part thereof and illustrate specific embodiments of the invention. Numbers in brackets or in bold linked to certain elements illustrate the elements in question as an example, without limiting the elements. It is to be understood that other embodiments may be used and structural or logical changes may be made without departing from the scope of the present invention. The following detailed description is not to be construed as limiting, and the scope of the present invention is defined by the appended claims.

Unless otherwise defined, all terms disclosed in the invention, including technical and scientific terms, have the meanings commonly understood by one of ordinary skill in the art. As further guidance, definitions are included for further explanation of terms used in the description of the invention.

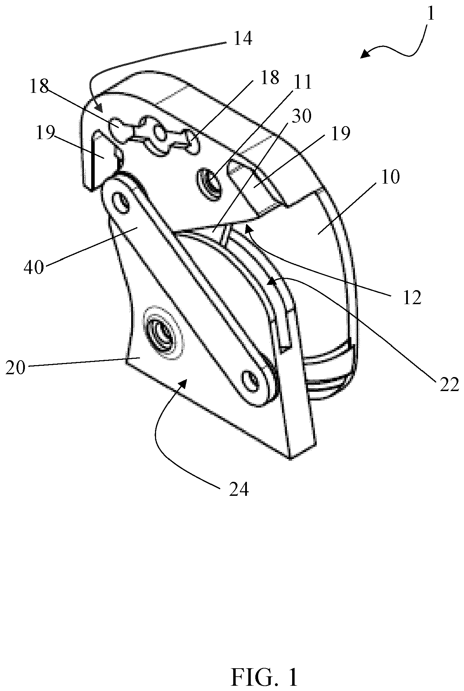

**Figure 1** shows a 3D side view of The hinge mechanism according to the invention. The hinge mechanism 1 is typically used in a knee orthosis 5 or knee brace, where the hinge mechanism 1 makes the connection between an upper part 6 of the knee orthosis 5 that is attached to the patient's upper leg and a lower part 7 of the knee orthosis 5 that is attached to the patient's lower leg. The hinge mechanism 1 will ensure that the bending movement of the patient's knee can occur in a correct and supportive manner. The hinge mechanism 1 consists of a first part 10 that can be connected to the upper part 6 of the knee orthosis 5. The hinge mechanism 1 further consists of a second part 20 that can be connected to the lower part 7 of the knee orthosis 5. In Figure 1 the hinge mechanism 1 is shown without the connection to the upper part 6 and the lower part 7. If the first part 10 of the hinge mechanism 1 is not permanently connected to the upper part 6 of the knee orthosis 5, the first part 10 is provided with a connecting mechanism (not shown) complementary to a connecting mechanism (not shown) of the upper part 6. If the second part 20 of the hinge mechanism 1 is not permanently connected to the lower part 7 of the knee orthosis 5, the second part 20 is provided with a connecting mechanism (not shown) complementary to a connecting mechanism (not shown) of the lower part 7. If the hinge mechanism 1 in that case needs to be connected to the upper 6 and lower 7 parts of the knee orthosis 5, the connecting mechanisms of the hinge mechanism 1 can be connected to the upper part 6 and lower part 7 in order to obtain a connection between the different parts.

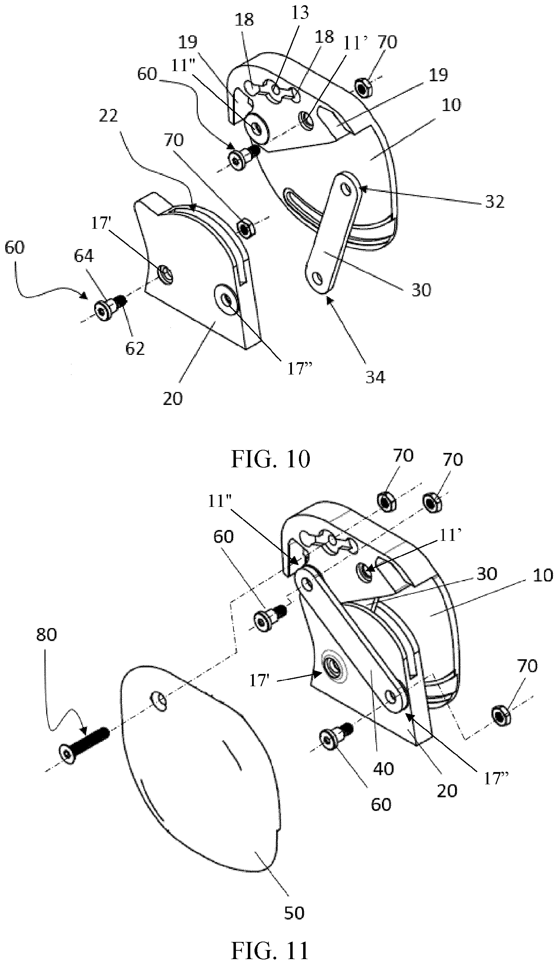

Various recesses 18, 19 are provided on a first side 14 of the first part 10. These recesses 18, 19 are provided to enable correct alignment of a cover plate 50 with respect to the first part 10. Moreover, the recesses 18, 19 ensure that the cover plate 50, after mounting on the first part 10 with complementary protrusions 52, 54 placed in the recesses 18, will move along with the rotational movement of the first part 10. In addition, an opening 13 is provided on the first side 14 of the first part 10, in which, after the cover plate 50 has been brought into the correct position via the protrusions 52, 54, it can be secured to the body via a bolt 80 (see Figure 11). first part 10.

Figure 1 shows a second part 20, whereby the front 24 of the second part 20, after mounting, comes to lie flush with the front 14 of the first part 10. For this purpose, the second part 20 is placed under the first part, so that the surfaces 14 and 24 are flush with each other.

A first coupling piece 30 ensures that a hinged connection between the first part 10 and the second part 20 is established. To make this possible, the first part10 and the second part 20 are provided with incisions 12, 22. The first coupling piece 30 can be inserted on a first side 32 into the incision 12 of the first part 10 and secured in a hinged manner, while the coupling piece 30 on the other side 34 is inserted into the incision 22 of the second part 20 and secured in a hinged manner.

Furthermore, a second coupling piece 40 is shown in Figure 1, wherein the second coupling piece 40 can be hingedly attached to the sides 14 and 24 of the first part 10 and the second part 20 respectively, thus also creating a hinged connection between the first part 10 and the second part 20 can be obtained. The second coupling piece 40 also ensures that the first 10 and the second 20 parts are secured with respect to each other.

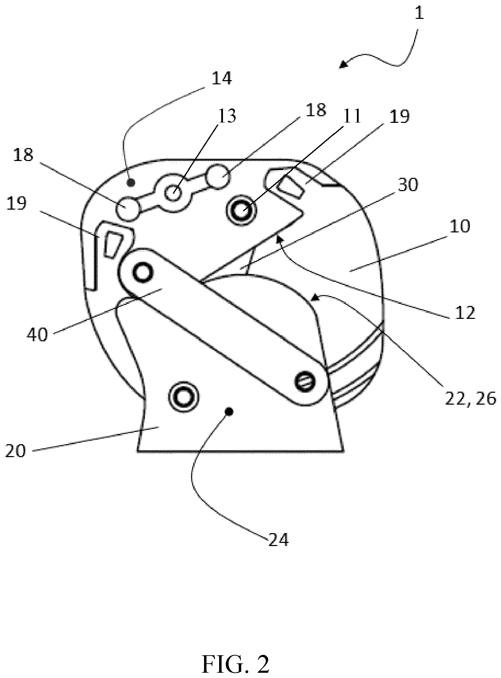

**Figure 2** shows a side view of the hinge mechanism 1 as shown in Figure 1, where it is clear that the second part 20 is placed under the first part 10, and where the first coupling piece 30 has been inserted into the incisions 12 and 22, while the second coupling piece 40 is placed on the front sides 14 and 24 of the first 10 and second 20 parts, respectively.

In both Figure 1 and Figure 2, the cover plate 50 has been omitted in order to clearly show the relative positions of the various parts.

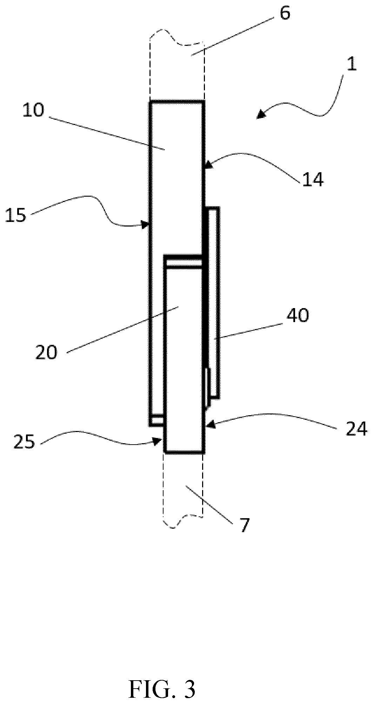

**Figure 3** shows a front view of the hinge mechanism 1 as shown in Figure 1 and Figure 2. This front view shows that the second part 20 is positioned under the first part 10, wherein the front side 14 of the first part 10 and the front side 24 of the second part 20 will lie in the same plane. It is also indicated in a broken line that the upper part 6 of the knee orthosis 5 is connected to the first part 10 of the hinge mechanism 1, while the lower part 7 of the knee orthosis 5 is connected to the second part 20 of the hinge mechanism 1.

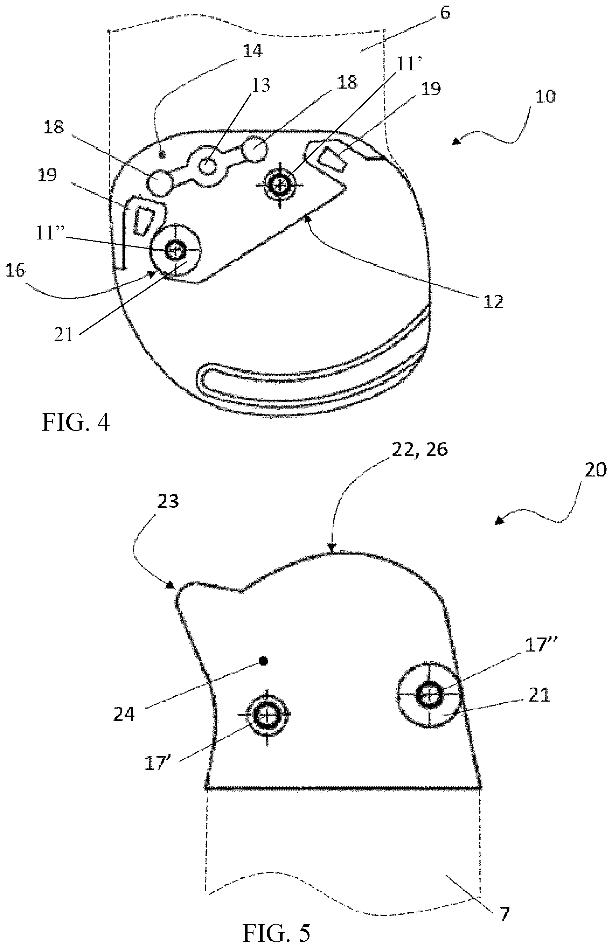

**Figure 4** shows a side view of a first part 10 of the hinge mechanism 1 that can be connected to an upper part 6 of a knee orthosis 5. Alternatively, it is possible that the first part 10 and the upper part 6 are made from one piece. The first part 10 is provided with a thickening in which the recesses 18 and 19 for the protrusions 52, 54 of the cover plate 50 are provided. Also in this thickening are the openings 11', 11" and 13, which pass through the thickening from one side 14 to the other side 15. Through the holes 11' and 11" bolts 60 can be inserted to attach the first 30 or the second 40 connector to the first part 10. A rounding 16 is provided at the bottom of the thickening, which is complementary to a rounded top 26 of the second part (see Figure 5). The incision 12 is also provided on the underside of the thickening, which makes it possible to insert a first side 32 of the first coupling piece 30 into the incision 12 so that the first coupling piece 30 can be hinged into the thickening of the first part 10 and through the opening 11' can be hingeably attached to the first part 10. A bolt 60 can also be inserted through the opening 11" to attach the second coupling 40 hingeably to the first part 10. In order to ensure the necessary freedom of movement of the second coupling piece 40 in relation to the first part 10, it is possible to fit a circle 21 between the second coupling piece 40 and the first part 10 before inserting the bolt through the intended opening into the second coupling piece and the hole 11".

A banana-shaped cutout is provided at the bottom of the first part 10. This banana-shaped cutout is provided to provide space for a bolt 60 (see Figure 5), whereby the bolt can be inserted into the cutout, so that a compact assembly is possible.

**Figure 5** shows a side view of a second part 20 of the hinge mechanism 1 that can be connected to a lower part 7 of a knee orthosis 5. Alternatively, it is possible that the second part 20 and the upper part 7 are made from one piece. A rounding 26, which is complementary to a rounded bottom 16 of the first part, is provided at the top of the second part 20 (see Figure 4). Also provided in the second part 20 are the openings 17' and 17" which run from one side 24 to the other side 25 through the second part. Through the holes 17' and 17" bolts 60 can be inserted to attach the first 30 or the second 40 connector to the second part 10. The incision 22 is also provided in the rounding 26, which makes it possible to insert a second side 34 of the first coupling piece 30 into the incision 22, so that the first coupling piece 30 fits into the second part 20 at the level of the second side 34 and through the opening 17' can be hinged to the first part 10. A bolt 60 can also be inserted through the opening 17" to attach the second coupling 40 hingeably to the second part 20. In order to ensure the necessary freedom of movement of the second coupling piece 40 in relation to the second part 20, it is possible to fit a rondel 21 between the second coupling piece 40 and the second part 20 before inserting the bolt through the opening provided into the second coupling piece and the opening 17". Furthermore, a stop 23 has been fitted at the top of the second part 20, which connects to the rondel 26. When the first part 10 moves relative to the second part 20 in a mounted condition, this stop 23 will limit the movement of the first part 10, so that a limited pivoting movement of the hinge mechanism 1 is possible.

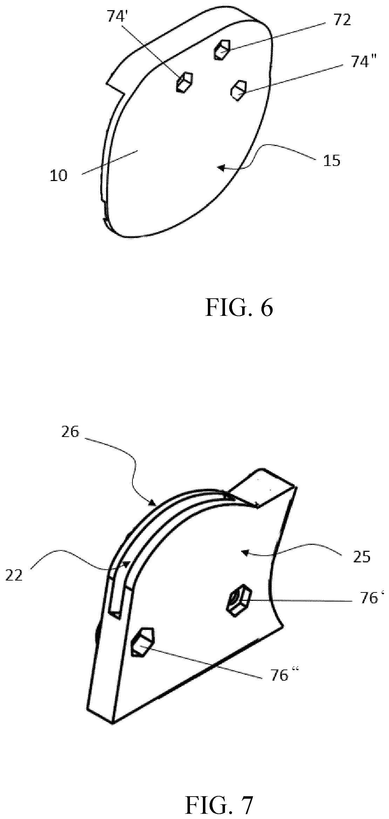

**Figure 6** shows a 3D side view of the first part 10 as shown in Figure 4. In Figure 6 the second side or the rear 15 of the first part 10 is visible, while in Figure 4 the first side or the front 14 of the first part 10 is visible. In this rear 15, some openings 72, 74' and 74" are visible; these openings 72, 74' and 74" have a shape complementary to a nut 70. When a nut 70 is inserted into these openings 72, 74' or 74", they will not be able to move relative to the first part 10 due to their complementary shape. The opening 72 is provided for the insertion of the nut 70, which together with the fastening means 80, for example a bolt 80, will attach the cover plate 50 to the first part 10. The opening 74" is provided for the insertion of the nut 70 which, together with a bolt 60, will attach the first side 40 of the first coupling piece 30 to the first part 10. The opening 74' is provided for the insertion of the nut 70 which, together with a bolt 60, will attach the first side 42 of the first coupling piece 30 to the first part 10.

**Figure 7** shows a 3D side view of the second part 20 as shown in Figure 5. In Figure 7 the second side or the rear 25 of the second part 20 is visible, while in Figure 5 the first side or the front 24 of the second part 20 is visible. In this rear side 25, some openings 76' and 76" are visible; these openings 76' and 76" have a shape complementary to a nut 70. When a nut 70 is inserted into these openings 76' or 76", they will not be able to move relative to the first part 20 due to their complementary shape. The opening 76' is provided for the insertion of the nut 70 which, together with a bolt 60, will attach the first side 34 of the first coupling piece 30 to the second part 20. The opening 76' is provided for the insertion of the nut 70 which, together with a bolt 60, will attach the second side 44 of the first coupling piece 40 to the second part 20. Figure 7 also shows the opening 22 that is provided in the top 26 of the second part 20, and into which the first coupling piece 30 can be inserted to be hingedly connected to the second part 20.

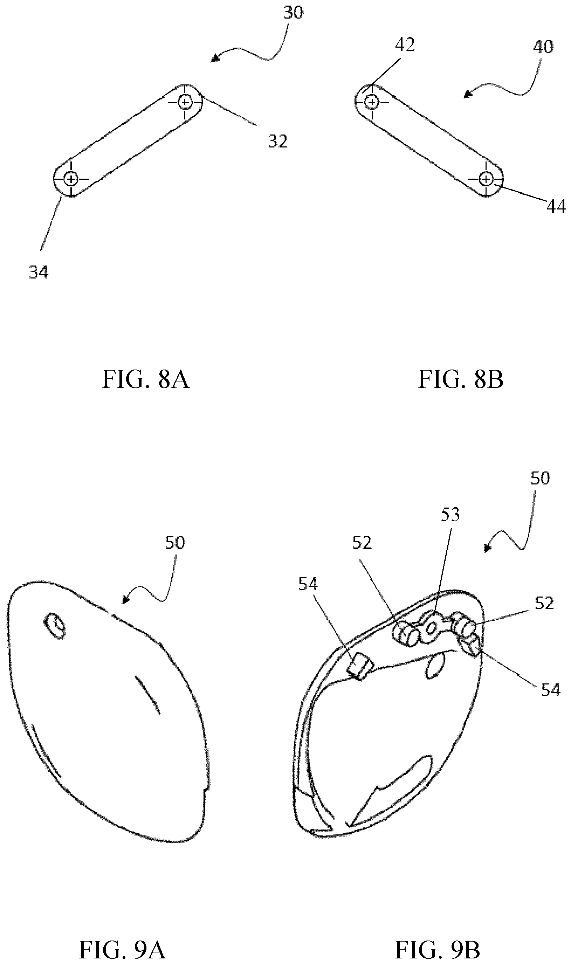

**Figure 8A** shows a side view of a first coupling piece 30 of the hinge mechanism 1 as shown in Figure 1. The coupling piece 30 is provided with an opening on a first side 32 through which a bolt 60 can be inserted. When mounting the hinge mechanism 1, the first side 32 will be inserted into the opening 12 of the first part 10 and can be hingedly connected to the first part 10 by means of a bolt 60 and nut 70 and through the openings 11', 74'. Furthermore, the coupling piece 30 is provided on a second side 34 with an opening through which a bolt 60 can be inserted. When mounting the hinge mechanism 1, the second side 34 will be inserted into the opening 22 of the second part 20 and can be hingedly connected to the second part 20 by means of a bolt 60 and nut 70 and through the openings 17', 76'.

**Figure 8B** shows a side view of a second coupling piece 40 of the hinge mechanism 1 as shown in Figure 1. The coupling piece 40 is provided with an opening on a first side 42 through which a bolt 60 can be inserted. The first side 42, when the hinge mechanism 1 is fitted by means of a bolt 60 and nut 70 and through the holes 11", 74" can be hingedly connected to the first part 10.

Furthermore, the coupling piece 40 is provided on a second side 44 with an opening through which a bolt 60 can be inserted. When mounting the hinge mechanism 1, the second side 44 will be hingedly connected to the second part 20 by means of a bolt 60 and nut 70 and through the openings 17', 76'. For the placement of the second coupling piece 40, it is possible to provide a washer 21 between the second coupling piece 40 on the one hand and the first side 14 of the first part 10 and the first side 24 of the second part 20.

**Figure 9A** shows a 3D front view of a cover plate 50 of the hinge mechanism 1 as shown in Figure 1. **Figure 9B** shows a 3D rear view of the cover plate 50 as shown in Figure 9A. A number of protrusions 52, 54 are provided on the rear side of the cover plate, which are complementary to the recesses 18, 19 provided in the thickening in the first part 10. When the cover plate 50 is to be mounted on the first part 10, the protrusions 52, 54 can be inserted into the respective recesses 18, 19. Moreover, the protrusions 52 are designed in such a way that they are connected to each other via an intermediate piece 53. In the thickening of the first part 10, the recesses 18 are also connected to each other via an additional recess, complementary to the intermediate piece 53.

By using the protrusions 52, 53, 54 and the recesses 18, 19, it is possible to connect the cover plate 50 to the first part 10 of the hinge mechanism 1 with only one bolt 80 and nut 70. As a result, when the first part 10 moves relative to the second part, the cover plate 50 always moves in the same way as the first part 10, regardless of the tightening of the nut 70 on the bolt 80.

**Figure 10** shows the exploded view of the hinge mechanism 1 as shown in Figure 1. Figure 10 shows how the bolts 60 fit into the respective openings 11', 17' and through the first 32 and second 34 sides of the first coupling piece 30. introduced. Also shown are the nuts 70 that connect the respective bolts 60 to the first part 10 and the second part 20. The bolts 60 are provided with a screw thread over a first length 62 in which the nut 70 can engage and are designed flat over a second length 64. The first 62 and the second length 64 are designed in such a way that the openings of the first coupling piece 30 are located at the level of the second flat length 64 after mounting the first coupling piece 30 in the first part 10 and the second part 20.

**Figure 11**shows the exploded view of the hinge mechanism shown in Figure 1, including the cover plate shown in Figures 9A and 9B; Figure 11 shows the way in which bolts 60 are inserted into the respective openings 11", 17" and through the first 42 and second 44 sides of the second connector 40. Also shown are the nuts 70 that connect the respective bolts 60 to the first part 10 and the second part 20. The bolts 60 are provided with a screw thread over a first length 62 in which the nut 70 can engage and are designed flat over a second length 64. The first 62 and the second length 64 are designed in such a way that the openings of the first coupling piece 40 are located at the level of the second flat length 64 after mounting the first coupling piece 40 in the first part 10 and the second part 20. Figure 11 also shows how the bolt 80 is inserted into the opening of the cover plate 50 and through the openings 13 and 72 of the first part. Bolt 80 is provided with screw threading over the entire length of bolt 80, but a similar embodiment such as bolt 60 is also possible.

Preferably, the method for mounting the hinge mechanism 1 consists of the following steps:
- inserting the side 34 of the first coupling piece 30 into the incision 22 of the second part 20.
- hingedly attaching the side 34 of the first coupling piece 30 to the second part 20 by fastening means. The fastening means are preferably a first bolt 60 and a first nut 70, whereby the bolt 60 passes through the opening 17' provided in the front 24 of the second part 20, the opening at the level of the side 34 of the first coupling piece 30 and the opening 76' is introduced at the rear 25 of the second part 20, and is connected to the second part 20 by means of the nut 70 in the opening 76'.
- hingedly attaching the first coupling piece 40 to the second part 20 by means of fastening means,. The fastening means are preferably a second bolt 60 and a second nut 70, whereby the bolt 60 is introduced through the opening 17" provided in the front 24 of the second part 20, the opening at the side 44 of the second coupling piece 40 and the opening 76" at the rear 25 of the second part 20, and is connected to the second part 20 by means of the nut 70 in the opening 76".
- inserting the side 32 of the first coupling piece 30 into the incision 12 of the first part 10.
- hingedly attaching the side 32 of the first coupling piece 30 to the first part 10 by means of fastening means. The fastening means are preferably a third bolt 60 and a third nut 70, whereby the bolt 60 passes through the opening 11' provided in the front side 14 of the first part 10, the opening at the level of the side 32 of the first coupling piece 30 and the opening 74' is introduced at the rear 15 of the first part 10, and is connected to the first part 10 by means of the nut 70 in the opening 74'.
- hingedly attaching the first coupling piece 40 to the first part 10 by means of fastening means. The fastening means are preferably a fourth bolt 60 and a fourth nut 70, whereby the bolt 60 passes through the opening 11" provided in the front 14 of the first part 10, the opening at the level of the side 42 of the second coupling piece 40 and the opening 74" is introduced at the rear 15 of the first part 10, and is connected to the first part 10 by means of the nut 70 in the opening 74".

In this way, the first part 10 is connected to the second part 20 and the first coupling piece 30 and the second coupling piece 40 will together form a hinge connection that makes the movement of the first part 10 relative to the second part possible.

It is further possible to connect a cover plate 50 to the first part 10 in order to shield the moving parts. The method for mounting the hinge mechanism 1 here also consists of the following steps:
- positioning at least one protrusion 52, 54 of the cover plate 50 relative to at least one complementary recess 18, 19 in the first part 10; and
- connecting the cover plate 50 to the first part 10 by means of fastening means. The fastening means are preferably a first bolt 80 and a fifth nut 70, whereby the bolt 80 passes through the opening provided in the cover plate 50, the opening 13 at the front side 14 of the first part 10 and the opening 72 at the level of the rear 15 of the first part 10, and is connected to the first part 10 by means of the nut 70 in the opening 72.

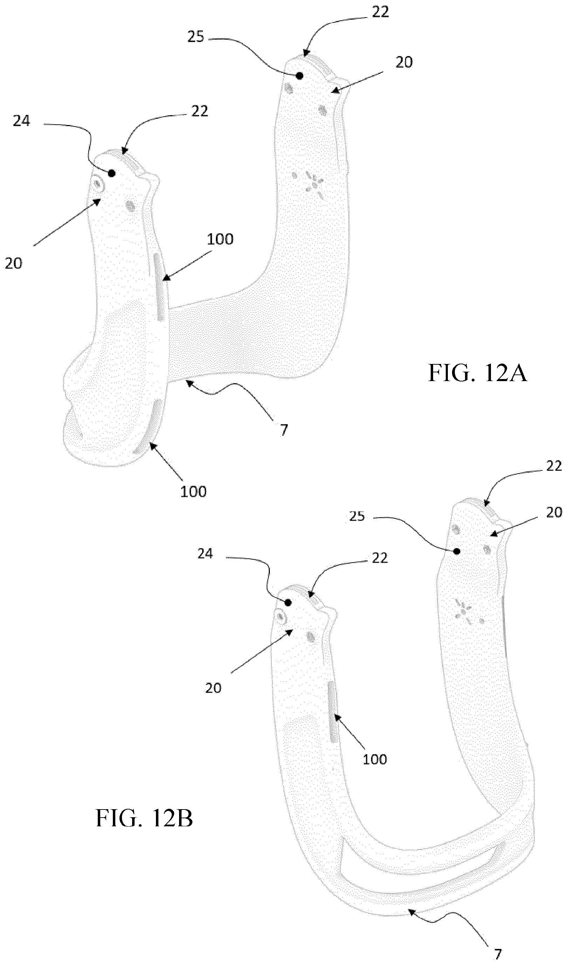

**Figure 12A** shows a 3D side view of the second part of the hinge mechanism 1 connected to the lower part 7 of a knee orthosis. In the representation of Figure 12A, the second parts 20 of a left and right hinge mechanism and the lower part 7 are permanently connected to one another. Preferably, the second parts 20 and the lower part 7 are formed via 3D printing and from one piece. Forming this part from a whole and via 3D printing has the advantage that the whole does not have fracture lines, which results in a strong whole.

Openings 100 are also provided in the lower part 7. It is possible to place through these openings 100 a strap (not shown) which can be placed around the patient's lower leg, thus firmly attaching the lower part 7 to the patient's lower leg. A well-known way is to use a hook-and-loop tape where the hook-and-loop tape can stick to itself and can be easily attached and released.

In the case of Figure 12A, the lower part 7 should be placed at the back of the patient's lower leg.

**Figure 12B** shows a 3D side view of an alternative as shown in Figure 12A. The embodiment of Figure 12B is very similar to the embodiment of Figure 12A, with the difference that the lower part 7 is folded forward, while the lower part 7 of Figure 12A is folded backward. In the case of Figure 12B, the lower part 7 should be placed at the front of the patient's lower leg. In the embodiment of Figure 12B, an opening is provided in the lower part 7. By providing an opening in one or more strategic places, it is possible to make the knee orthosis 5 as light as possible, which should improve wearing comfort for the patient.

The choice to use the embodiment of Figure 12A or Figure 12B depends on the wishes of the patient and the injury to the patient's knee joint. In this way it is possible to increase the wearing comfort that the patient experiences when the patient uses the knee orthosis 5.

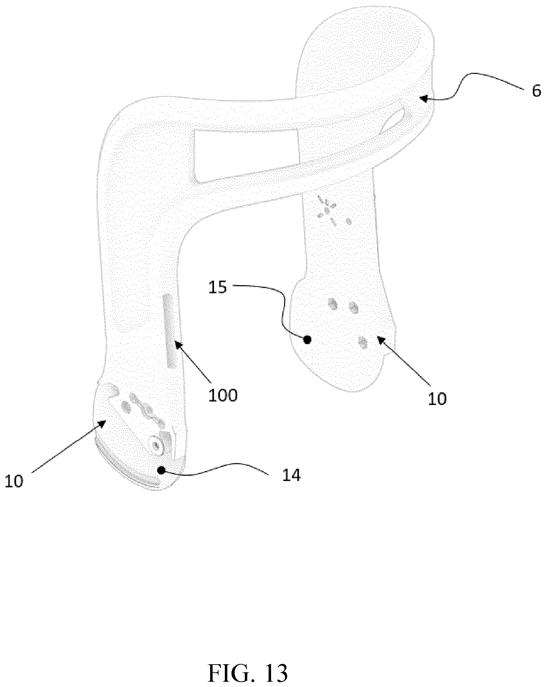

**Figure 13** shows a 3D side view of the first part 10 of the hinge mechanism connected to the upper part 6 of a knee orthosis 5. In the representation of Figure 13, the first parts 10 of a left and right hinge mechanism 1 and the upper part 6 are permanently connected to each other. Preferably, the first parts 10 and the upper part 6 are formed via 3D printing and in one piece. Forming this part from a whole and via 3D printing has the advantage that the whole does not have fracture lines, which results in a strong whole.

At least one opening 100 is also provided in the upper part 6. It is possible to place through these openings 100 a strap (not shown) which can be placed around the patient's lower leg, thus firmly attaching the upper part 6 to the patient's upper leg. A well-known way is to use a hook-and-loop tape where the hook-and-loop tape can stick to itself and can be easily attached and released.

In the case of Figure 13, the upper part 6 should be placed at the front of the patient's thigh. In the embodiment of Figure 13, an opening is provided in the upper part 6. By providing an opening in one or more strategic places, it is possible to make the knee orthosis 5 as light as possible, which improves wearing comfort for the patient.

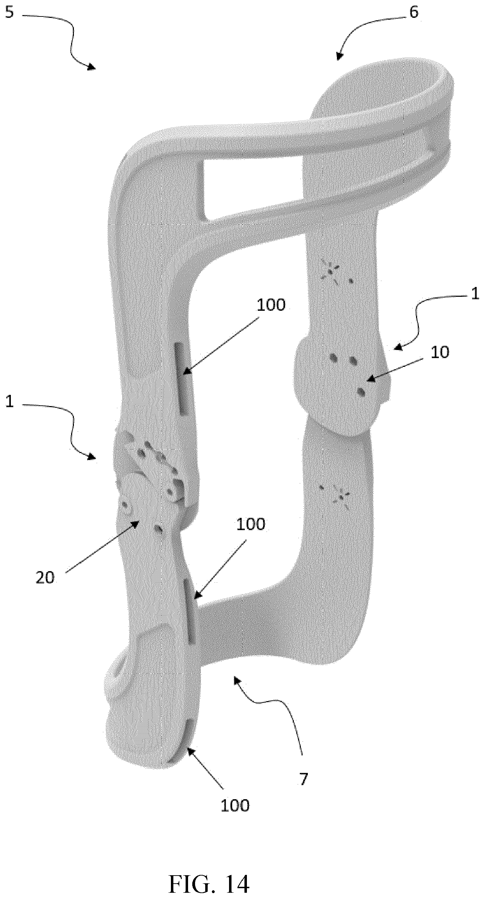

**Figure 14** shows a 3D representation of a knee orthosis 5, wherein the first parts 10 and the second parts 20 of the left and right hinge mechanism are positioned relative to each other. The lower part 7 of the knee orthosis 5 as shown in Figure 14 is similar to the lower part 7 as shown in Figure 12A, wherein the lower part 7 must be secured behind the patient's lower leg. The upper part 6 as shown in Figure 14 is similar to the upper part as shown in Figure 13 while the upper part 6 should be secured in front of the patient's upper leg.

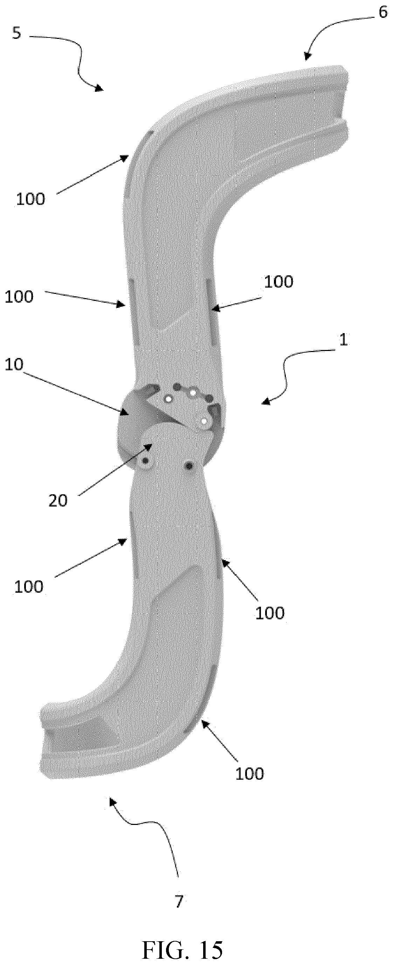

**Figure 15** shows a side view 90 of the knee orthosis 5 shown in Figure 14. From this side view it can be noted that the openings are provided throughout the entire lower 7 and upper portions 6 of the knee orthosis 5, so that the straps can be placed through these openings and will exert similar pressure on the patient's leg.

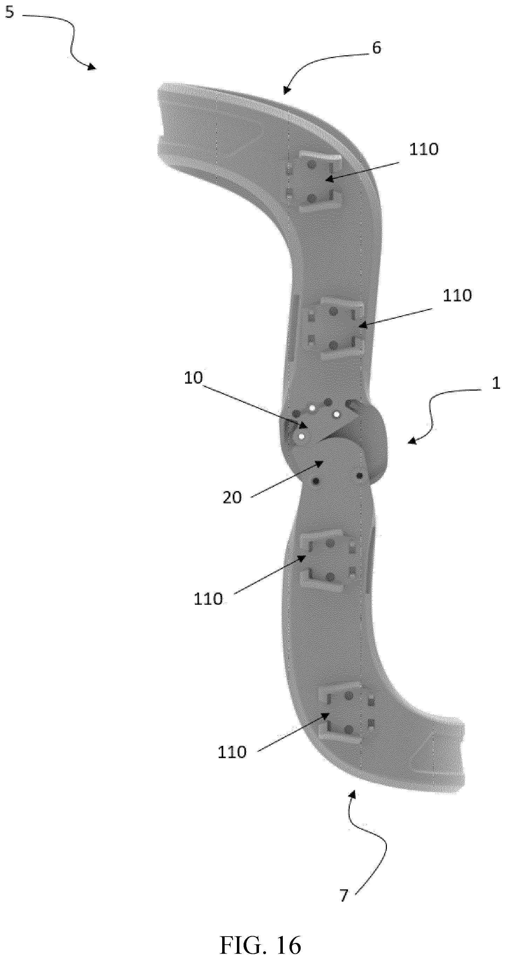

**Figure 16** shows another side view 95 of the knee orthosis 5 shown in Figure 14. Figure 16 shows that guide profiles 110 are provided on this side 95 of the knee orthosis. The purpose of these guide profiles 110 is to guide the attached straps through the openings 100 and to keep them in place, so that the straps cannot shift during the movement of the patient's leg, and will thus hold the knee orthosis 5 in the intended position relative to the knee joint.

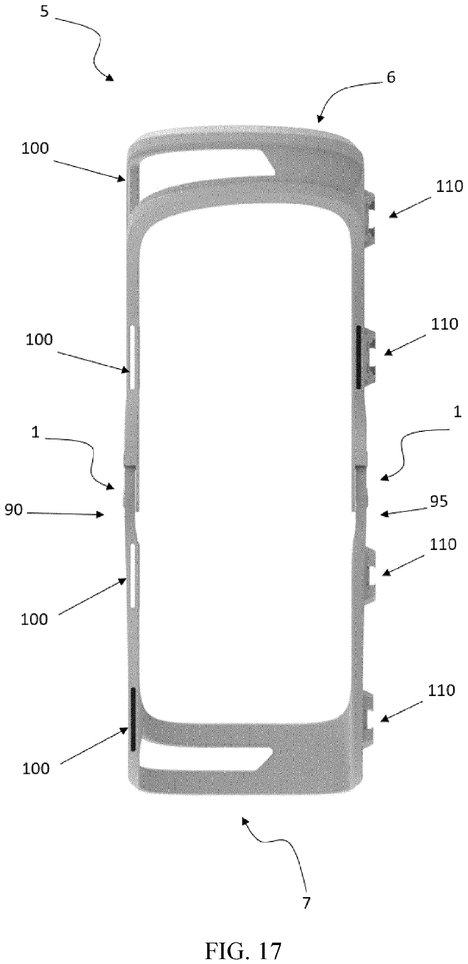

**Figure 17** shows a front view of the knee orthosis as shown in Figure 14, where the openings provided in the upper 6 and lower 7 parts are visible. It can also be noted from this figure that the assembly of the hinge mechanism 1 is compact, whereby the first side 14 of the first part 10 and the first side 24 of the second part 20 of the respective left and right hinge mechanism come to lie flush with each other.

It is clear that further variants and combinations are possible without departing from the scope of protection as defined in the claims.

## Claims

1. A hinge mechanism (1) for knee orthosis (5), wherein the hinge mechanism consists of:
- a first part (10) connected to an upper part (6) that can be placed above a knee joint,
- a second part (20) connected to a lower part (7) that can be placed below the knee joint,
- a first coupling piece (30), hingedly connected to the first part (10) and the second part (20), and
- a second coupling piece (40), hingedly connected to the first part (10) and the second part (20);
**characterized in that** the first part (10) and the second part (20) are provided with incisions (12, 22), wherein the first coupling piece (30) is inserted into the incision (12) on a first side (32) and hingedly attached to the first part (10), and wherein the coupling piece (30) on the other side (34) is inserted into the incision (22) and hingedly attached to the second part (20).

2. The hinge mechanism according to claim 1, wherein the hinge mechanism further consists of a cover plate (50), and wherein the second coupling piece (40) is positioned between the cover plate (50) on the one hand, and the first part (10) and the second part (20), on the other.

3. The hinge mechanism according to claim 2, wherein the cover plate (50) is firmly connected to the first part (10).

4. The hinge mechanism according to claim 3, wherein the cover plate (50) is provided with at least one protrusion (52, 54), and wherein at least one recess (18, 19) is provided in the first part (10), such that, after mounting the cover plate (50) on the first part (10), the at least one protrusion (52, 54) of the cover plate (50) engages in the at least one recess (18, 19) of the first part (10).

5. The hinge mechanism according to claim 4, wherein the at least one protrusion (54) has a wedge shape and wherein the at least one recess (19) has a complementary wedge-shaped opening.

6. The hinge mechanism according to any of the preceding claims, wherein the first part (10) is positioned relative to the second part (20), such that the first side (24) of the second part (20) is flat with respect to the first side (14) of the first part (10).

7. The hinge mechanism according to any of the preceding claims, wherein the top side (26) of the second part (20) is rounded, and wherein the first part (10) is provided with a rounding (16) that is complementary to the rounded top side (26).

8. The hinge mechanism according to claim 7, wherein the incision (22) is provided in the rounded top (26) of the second part (20).

9. The hinge mechanism according to any of the preceding claims, wherein the first coupling piece (30) and the second coupling piece (40) are hingedly connected to the first part (10) and the second part (20) by means of a bolt (60) and a nut (70).

10. The hinge mechanism according to claim 9, wherein the bolt (60) is threaded over a first length (62) and is flat over a second length (64).

11. The hinge mechanism according to claim 9 or 10, wherein the second side (15) of the first part (10) and the second side (25) of the second part (20) are provided with recesses, and wherein the recess is complementary to the shape of the nut (70).

12. Method for mounting the hinge mechanism according to any of the preceding claims, wherein the method consists of the following steps:
- inserting the first coupling piece (30) into the incision (22) of the second part (20).
- hingedly attaching the first coupling piece to the second part by means of fastening means (60, 70),
- hingedly attaching the second coupling piece (40) to the second part (20) by means of fastening means (60, 70),
- inserting the first coupling piece (30) into the incision (12) of the first part (10).
- hingedly attaching the first coupling piece (30) to the first part (10) by means of fastening means (60, 70), and
- hingedly attaching the second second coupling piece (40) to the first part (10) by means of fastening means (60, 70).

13. The method according to claim 12, wherein the method further consists of the steps:
- positioning at least one protrusion (52, 54) of a cover plate (50) relative to at least one complementary recess (18,, 19) in the first part, and
- connecting the cover plate (50) to the first part (10) by means of a fastening means (70, 80).

14. A knee orthosis (5), wherein the knee orthosis (5) is provided with two hinge mechanisms (1) according to any of claims 1 to 11, and wherein the first hinge mechanism (1) is positioned on a first side (90) of the knee orthosis (5) between the upper part (6) and the lower part (7) of the knee orthosis (5), and wherein the second hinge mechanism (1) is positioned on a second side (95) of the knee orthosis (5) between the upper part (6) and the lower part (7) of the knee orthosis (5).

15. A knee orthosis (5) according to claim 14, wherein the first part (10) of the first hinge mechanism (1), the upper part (6) and the first part (10) of the second hinge mechanism (1) are formed in one piece, and wherein the second part (20) of the first hinge mechanism (1), the lower part (7) and the second part (20) of the second hinge mechanism (1) are formed in one piece.

16. A knee orthosis (5) according to claim 14 or 15, wherein the parts of the knee orthosis are formed by 3D printing.
